(19) **Europäisches Patentamt**
**European Patent Office**
**Office européen des brevets**

(11) **EP 4 346 387 B1**

(12) **EUROPEAN PATENT SPECIFICATION**

(45) Date of publication and mention
of the grant of the patent:
**30.04.2025 Bulletin 2025/18**

(21) Application number: **22727515.3**

(22) Date of filing: **18.05.2022**

(51) International Patent Classification (IPC):
**G01N 33/543** (2006.01)    **A01J 5/013** (2006.01)
**G01N 33/04** (2006.01)

(52) Cooperative Patent Classification (CPC):
**A01J 5/0131; A01J 5/0135; G01N 33/04;
G01N 33/54388**

(86) International application number:
**PCT/SE2022/050486**

(87) International publication number:
**WO 2022/250593 (01.12.2022 Gazette 2022/48)**

(54) **DETERMINATION OF HAPTOGLOBIN QUANTITY IN MILK**

BESTIMMUNG DER HAPTOGLOBINMENGE IN MILCH

DÉTERMINATION DE QUANTITÉ D'HAPTOGLOBINE DANS DU LAIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.05.2021 SE 2150656**

(43) Date of publication of application:
**10.04.2024 Bulletin 2024/15**

(73) Proprietor: **DeLaval Holding AB
147 21 Tumba (SE)**

(72) Inventors:
• **CHRISTENSEN, John M
147 21 Tumba (SE)**
• **DOOLEWEERDT RASMUSSEN, Camilla
147 21 Tumba (SE)**
• **TRIER HALD, Jonas
147 21 Tumba (SE)**

(74) Representative: **DeLaval International AB
Intellectual Property Support/Legal Affairs
P.O. Box 39
147 21 Tumba (SE)**

(56) References cited:
**WO-A1-2014/055011        US-A- 5 047 351
US-A1- 2018 180 632**

• **BRADY N ET AL: "Development of a Lateral Flow
Point of Care Test for the Rapid Detection and
Measurement of Haptoglobin in Bovine Milk",
PROCEEDINGS OF THE BRITISH MASTITIS
CONFERENCE 2016; 2ND NOVEMBER 2016,
WORCESTER, WORCESTERSHIRE, SIXWAYS,
WORCESTER, 1 January 2016 (2016-01-01),
pages 69 - 70, XP009536674**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to determination of haptoglobin in milk. The invention takes into account that determination of haptoglobin quantity may be influenced by blood being present in the milk and the invention provides a device configured to adjust a value of haptoglobin quantity provided by a haptoglobin sensor device based on blood detection in the milk.

BACKGROUND OF THE INVENTION

Milk production in animals, especially cows have been optimized severely over the years through efficiency gain and selective breeding. The higher milk yield requires more feed but also healthy cows and good farm management to be maintained. Until now the industry has mainly been focusing on selective breeding and genetic breeding in relation to milk yield. However, increasing focus on disease detection and treatments is starting to be of more interest. Monitoring of parameters in the milk from cows could be of support herefore.

Several diseases may affect the milk yield and health status of the cows such as mastitis acute and chronic, ketosis and metabolic disorders, metritis and other reproductive diseases, stress and lameness. Mastitis is a common disease which is an infection in the mammary gland. The consequences are low milk yield and quality. Treatments are based on antibiotics and can be with limited success. Ketosis is a metabolic disorder, which happens the first months after calving. This is often treated with propylene glycol and feed containing high amount of starch (and other sugar derivatives). Metritis is an infection in the uterine. Such infection takes place in the most sensitive part of the lactation with signs of anorexia, depressed mood, reduced appetite, and feed intake with the short-term result that the lactational milk yield become significantly lower than in no metritis cows. Additional effects on the longer term are "low success" rate on inseminations, increasing the open days, which in return extends that time with low milk yield, before the next calving time. Stress affect the well-being of the cow and can give abortions, low milk yield and low success rate of inseminations. Lameness is often caused by laminitis, claw disease, digital dermatitis, and foot rot. This limits the cows ability to move around and hereby access to feed, water and the general welfare of the cow.

Diseases such as metritis, mastitis, lameness and ketosis can be so severe that the outcome is that the cow is set to be culled. Almost all diseases impact profitability on the farm and animals welfare. Optimal treatments and prevention methods for the above-mentioned diseases is thus, of high importance. Further, as herds becoming bigger and bigger, less human monitoring of the individual cow may require sensor systems that can identify and point out the cow with an health issue.

For a long time it has been generally known that early detection of diseases increases the chances of effective treatment. Therefore, there is an incentive to increase on farm monitoring and testing. To do this many systems have been developed. Automatic health and reproduction monitoring are emerging to improve efficiency, labor use and animal welfare in a sustainable way.

The mentioned diseases trigger an early defense response of the innate immune system affecting the expression of several proteins. One of these is haptoglobin. Haptoglobin is positively upregulated as a cause of infections and is generally low in healthy cows why it constitutes a good disease marker as a response to infection and inflammation. Haptoglobin's main biological function is to bind free hemoglobin with high affinity to prevent loss of iron after intravascular hemolysis. Haptoglobin is present 24-48 hours after infection and has been demonstrated to be present in milk. Furthermore, studies show that the amount of haptoglobin can be correlated to clinical disease. Haptoglobin is a general health marker. If haptoglobin is used to differentiate between diseases it must be combined with other biomarkers. Haptoglobin can be used to measure the severity of the disease. Haptoglobin can thus be used as a marker for acute vs chronic mastitis, metritis, lameness, respiratory disease and as a general health marker. Thus, haptoglobin could be a rapid, precise and easy diagnostic tool to evaluate the effect of changes in farm management and as a tool to diagnose and obtain a more precise treatment of diseased dairy cows.

Hence, an easy available measurement system for potential on-site measurement of haptoglobin would be advantageous, and in particular, a reliable system for quantitatively measuring of haptoglobin to determine the present health status of milk-producing animals would be advantageous.

US2018/180632A1 discloses an apparatus for monitoring the state of health of a dairy cow, omprising: (a) means to take a milk sample from the dairy cow; (b) means to measure the concentration of haptoglobin as biomarker in the milk sample; (c) means to compare the measured concentration from (b) with a reference value of the measured biomarker.

OBJECT OF THE INVENTION

In particular, it may be seen as an object of the present invention to provide an analysing unit, preferably comprised in a milking arrangement for easy and reliable measurement of haptoglobin in milk that solves the above mentioned problems and provides the farmer with a readily available tool for establishing the health conditions of his animals.

SUMMARY OF THE INVENTION

Thus, the above described object and several other objects are intended to be obtained with the invention according to claim 1 by providing a milking arrangement comprising a milking machine for milking an animal, wherein the milking arrangement comprises

- an analysing unit configured to determining a value of haptoglobin quantity in a milk sample taken from milk harvest by the milking machine, said analysing unit comprising

  - a blood detection sensor device configured to detect the presence of blood in said milk harvest by the milking machine,
  - a haptoglobin sensor device configured to provide a value of haptoglobin quantity in said milk sample, and
  - a processor;

  wherein the processor being configured to adjusting said provided value of haptoglobin quantity based on the blood detection in the said milk.

The invention is particularly, but not exclusively advantageous for obtaining an adjusted value of haptoglobin being, at least potentially, more accurately determined, and thus to obtain a measurement of haptoglobin quantity of the milk on the farm.

BRIEF DESCRIPTION OF THE FIGURES

The present invention and in particular preferred embodiments thereof will now be described in more details with reference to the accompanying figures. The figures show ways of implementing the present invention and are not to be construed as being limiting to other possible embodiments falling within the scope of the attached claim set.

Figure 1 schematically illustrates a milking arrangement according to a preferred embodiment of the invention,
Figure 2 schematically illustrates a dry stick used in connection with preferred embodiments of the present invention to determine a value of haptoglobin quantity,
Figure 3A-C show the principle of the dry stick used in connection with preferred embodiments of the present invention to determine a value of haptoglobin quantity,
Figure 4 schematically illustrates a milking arrangement according to another preferred embodiment,
Figure 5 shows experimental results and algebraic relationship between provided value of haptoglobin quantity and provided value of blood quantity. In fig. 5, linear regression plot of measurements of lateral flow stick on different combinations of haptoglobin and blood in fresh milk. The lines illustrate regression lines.

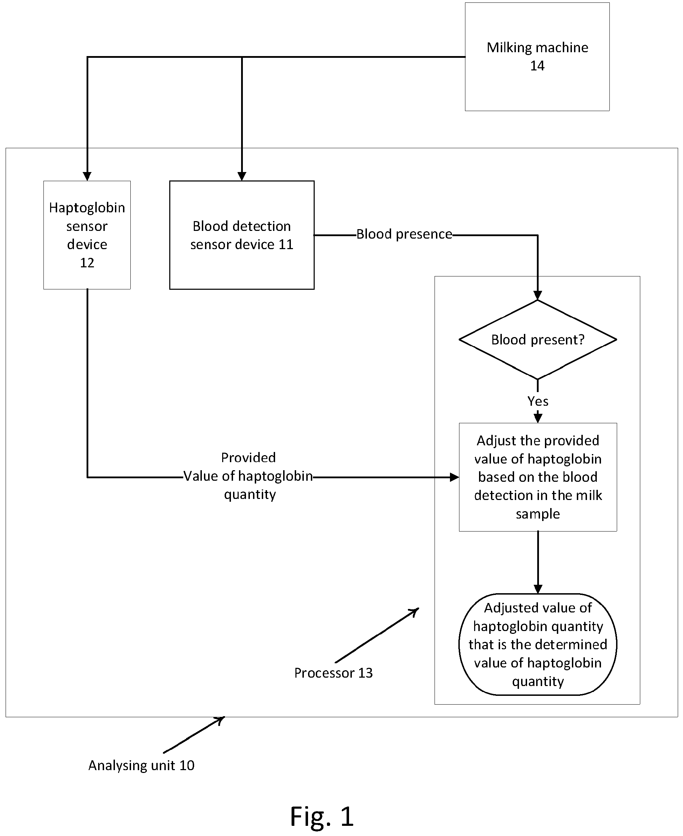

Reference is made to fig. 1 schematically illustrating a preferred embodiment of a milking arrangement. Elements of the milking arrangement are illustrated as boxes, and it is to be emphasised that the indicated boxes are not limiting for other ways implementing the milking arrangement. Accordingly, the box illustrating the analysing unit 10 in fig. 1, does not imply, e.g., that the sensors and processor must be embodied as a single unit, but relates merely to logical connections between the elements. In addition, the milking arrangement may comprise further elements than illustrated.
The milking arrangement comprises a milking machine 14 for milking an animal. The animal is preferably a cow, but the invention may be used for other milk producing animals. "Animal" may be any arbitrary type of domesticated female milk producing and/or meat producing mammal, such as cow, goat, sheep, horse, camel, primate, dairy buffalo, donkey, yak, etc. The milking machine may be part of a robotic milking station, rotary milking parlour or other milking parlours which harvest milk from the animal.
The milking arrangement is configured to draw a milk sample (not illustrated in fig. 1) typically of a known volume from milk being milked from the animal and to apply at least a part the milk sample to a haptoglobin sensor device 12.
As illustrated the milking arrangement comprises an analysing unit 10 configured to determine a value of haptoglobin quantity in the milk sample taken from milk harvest by the milking machine 14. Comprised in the analysing unit 10 is a blood detection sensor device 11 configured to detect the presence of blood in the milk harvest from the milking machine. As disclosed herein, the blood detection may be an in-line detection where no milk sample is taken. By in-line is typically meant that the sensor 11 is situated to detect in a flow-through system through which milk flows, such as in a milk line. However, the invention is not limited thereto and blood detection may be carried out on the basis of a milk sample. The blood detection sensor device 11 produces a signal representative of the blood detected. The detection of blood may be a Boolean value representing the two situations of either blood being present or not in the milk or (as will be disclosed in details below) a value of blood quantity, e.g. provided as ppm or percentage, in the milk. When the blood detection sensor device 11 is configured to provide a value representing blood quantity in the milk, the presence of an amount blood may translate into the Boolean value of blood being present.

Accordingly, the milk for which blood is to be detected and a value of haptoglobin quantity is to be determined is preferably milk harvest by the milking machine. However, the invention may also be used in connection with one or more fractions of the milk or even processed milk.

Further, a haptoglobin sensor device 12 is comprised in analysing unit 10. This haptoglobin sensor 12 is configured to provide a value of haptoglobin quantity in the milk sample. By haptoglobin quantity is typically meant either a concentration of haptoglobin or an absolute value of the amount of haptoglobin determined.

As disclosed herein, it has been found that blood in milk to be analysed with regard to the amount of haptoglobin present in the milk sample may interfer the value of haptoglobin provided by the haptoglobin sensor device. Such interference is suggested to be dealt with by the present invention by adjusting the provided value of haptoglobin quantity based on the blood detection.

As adjustment of the provided value of haptoglobin quantity typically involves some decision-making and/or arithmetic calculations a processor 13 is configured to perform such actions. The processor 13 is typically configured to performing such actions by comprising a software which when executed by the processor 13 performs the actions. Thus, one operation performed by the processor 13 is to adjust the provided value of haptoglobin quantity based on the blood detection in the milk, and to this the processor has software which when executed obtains both the value of haptoglobin quantity from the haptoglobin sensor device 12 and the result provided by the blood detection sensor device 11 and adjust the provided value of haptoglobin quantity based on the blood detection.

As illustrated in fig. 1, actions carried out by the processor may involve as an initial step to determine whether blood is present and adjust haptoglobin quantity if blood is present. As illustrated in fig. 1, the processor 13 obtains the value of haptoglobin quantity from the haptoglobin sensor device 12 and determines whether blood is present in the milk. If blood is present, subsequent steps comprise that the processor 13 obtains the value of haptoglobin quantity and adjust the value of haptoglobin quantity based on the blood detection.

It is noted, that the decisive step "Blood present?" shown in the fig. 1 may not need to be executed as such, since if an amount of blood in milk is determined, this automatically qualifies as blood being present so that there may not be a need to question whether or not blood is present. However, as detailed below, the decisive step "Blood present?" may be relevant, as the adjustment may involve discarding the provided value of haptoglobin.

Different approaches pertaining to adjusting the provided haptoglobin value are proposed by the present invention, and in one such approach the adjustment of provided value of haptoglobin quantity is to discard the value of haptoglobin quantity if blood is detected in the milk. By discard is typically meant that no value of haptoglobin quantity is assigned to have been provided or that the provided value of haptoglobin is marked as invalid or defective. This may have the advantage that a high degree of reliability can be assigned to a provided value of haptoglobin quantity as the interference on the provided value may in essence have been eliminated due to discarding of values of haptoglobin quantities provided for samples containing blood.

In some embodiments, values of haptoglobin quantities provided may be discarded if blood is detected the milk sample. It has, however, been found in connection with the present invention, that some experiments suggest that smaller amounts of blood in the milk has less interference on the provided haptoglobin value than larger amounts and that such correlation may e.g. be used to adjust the provided value of haptoglobin quantity.

In this and other regards, blood detection in milk typically involves determining a value of blood quantity in the milk. Also for the blood quantity is typically meant either a concentration of blood or an absolute value of the amount of blood. This determination of blood quantity is providing by use of the blood detection sensor device 11 that is configured for this purpose. It is noted that a blood detection sensor device 11 configured to determine a value of blood quantity in milk may also be used to determine the presence of blood in milk as such presence can be identified by the sensor device.

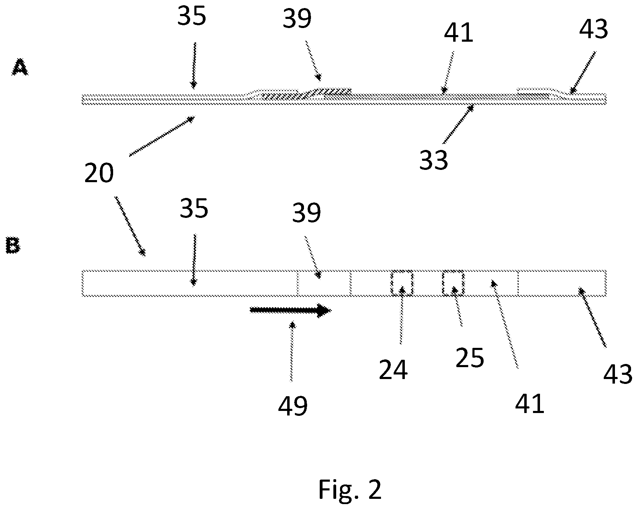

The interference on the provided haptoglobin quantity has been found to be profound in relation to a haptoglobin sensor device 12 involving the use of a dry stick 20, such as a lateral flow assay. One such dry stick is schematically illustrated in fig. 2. As illustrated the dry stick 20 has a test line 24 and a control line 25. It is noted that "line" is not to be interpreted narrowly in the sense of a mathematical definition where a line has no thickness. On the contrary and as also shown in fig. 2, the test line 24 and control line 25 both have a width.

Milk is applied to the dry stick, typically at end thereof, and due to the configuration of the dry stick milk is drawn laterally in the dry stick resulting in a colouring of the test line and control line with a colour intensity correlated with, inter alia, the amount of haptoglobin present in the milk. Such colouring is herein also referred to as a signal. The test line 24 may in some embodiment be optional.

In embodiments involving a dry stick (a dry stick according to the invention will be described below), the haptoglobin sensor device 12, comprising the dry stick, may have an optical device 19 that is configured to optically read a test signal at the test line 24 of the dry stick 20. As this test signal is correlated with the value of haptoglobin quantity, the test signal can be translated into a value of haptoglobin, e.g. by relating an actual test signal to signals obtained on the basis of milk samples with known amounts of haptoglobin as will be disclosed below.

The determination of the value of haptoglobin quantity is in preferred embodiments carried out by the processor 13 receiving the test signal and optionally a control signal. The processor 13 is configured to determine the provided value of haptoglobin quantity based on the received test signal. The processor 13, in general, has software allowing it to translate the test signal into a provided value of haptoglobin which software implements a correlation between test signal and value of haptoglobin quantity or performs a look-up in a database storing corresponding test signals and haptoglobin quantities. Examples on these are provided below.

In situations where the dry stick has a control line, the optical device may be configured to optically read also a control signal at the control line. Embodiments of dry sticks will be detailed here below.

Preferably, the dry stick is a competitive lateral flow stick configured to measure haptoglobin in a milk sample, said dry stick comprises:

- a base pad capable of allowing lateral flow of fluid there through;

  - comprising a labelled-control conjugate and a labelled-conjugate diffusibly arranged herein, wherein said labelled-conjugate binds haptoglobin, and wherein a complex is formed between said labelled-conjugate and said haptoglobin when said dry stick is in use;

wherein said base pad further comprises

- a test line comprising immobilised target analyte, wherein said immobilised target analyte binds to said labelled-conjugate when not in said complex; and
- a control line, which is spaced from said test line, and which comprises control analyte capable of binding to said labelled-control conjugate.

It is noted, that the control line is an optional feature. However, the below description has been made with reference to dry stick comprising both the control line and the test line.

By competitive lateral flow stick is to be understood that the dry stick is capable of allowing a lateral flow of liquid from one end of the dry stick to the other end of the dry stick as commonly known to the skilled person. By competitive is to be understood that the target analyte at the test line competes with haptoglobin in the sample for binding to the labelled-conjugate.

A dry stick as herein described may be implemented in a milking arrangement, potentially via a cassette, as described in e.g. WO 2020/251457.

*Base pad*

The dry stick comprises a base pad for allowing a lateral flow to take place. In its most simple form the base pad only comprises one module. However, the base pad may also comprise more modules, which can be of different materials depending on the specific purpose of the module. In an embodiment, said base pad comprises at least two modules. Hereby is to be understood that the base pad may comprise two or more modules.

In a further embodiment, the base pad comprises two modules being a membrane and a reagent pad. In a further embodiment, said membrane or at least a part hereof is downstream of said reagent pad.

In a further embodiment, the base pad comprises three modules being a membrane, a sample pad and a conjugate pad. In a further embodiment, said membrane or at least a part hereof is downstream of said conjugate pad and said conjugate pad or at least a part hereof is downstream of said sample pad.

In a further embodiment, a module is a reagent pad capable of receiving said milk sample. Hence, the base pad would comprise a module being a reagent pad. In an even further embodiment, said reagent pad comprises at least two modules being a sample pad and conjugate pad. In a further embodiment, the sample pad and the conjugate pad are partly overlapping. In one embodiment, the base pad comprises at least two modules, wherein one of said at least two modules is a sample pad capable of receiving said milk sample. If the reagent pad is one module, the sample pad and conjugate pad will be contained in this module and the milk sample will be received in the reagent pad.

In the present context, "reagent pad" relates to one or more pads comprising the labelled-control conjugate and the labelled-conjugate, which are both diffusible arranged in the reagent pad. The milk sample would also be applied to the reagent pad. In one embodiment, the reagent pad comprises a sample pad and a conjugate pad.

The material used for the reagent pad may be selected from the group of a nitrocellulose membrane, a cellulose, a polymer such as nylon, a polyvinylidene fluoride or latex, glass fibres, woven fibres, non-woven fibres and a chromatographic gel membrane.

In the present context, "sample pad" relates to a pad in the dry stick where the milk sample is applied to the dry stick and which provides a fast adsorption of the liquid sample and a fast and consistent release of the sample to the conjugate pad. The purpose of the sample pad is to collect sample. In some embodiments, it can be designed to withhold not wanted molecules present in the sample for these not to interfere with the functioning of the dry stick such as removing red blood cells, fat aggregates and large particles. The sample pad may be treated with a surfactant to release surface tension and quickly soak in the sample.

The material used for the sample pad may be selected from the group of a nitrocellulose membrane, a cellulose, a polymer such as nylon, a polyvinylidene fluoride or latex, glass fibres, woven fibres, non-woven fibres and a chromatographic gel membrane.

In the present context, "conjugate pad" relates to one or more pads comprising the labelled-control conjugate and the labelled-conjugate, which are both diffusible arranged in the conjugate pad.

The material used for the conjugate pad may be selected from the group of a nitrocellulose membrane, a cellulose, a polymer such as nylon, a polyvinylidene fluoride or latex, glass fibres, woven fibres, non-woven fibres and a chromatographic gel membrane.

In the present context, "diffusibly arranged herein" relates to the labelled-control conjugate and the labelled-conjugate being present in the base pad in a manner, which allows the labelled-control conjugate and the labelled-conjugate to be immobilised when the dry stick is in dry state and mobile when in moistened state i.e. when in use. Accordingly, the labelled-conjugate and the labelled-control conjugate will be maintained in the base pad e.g. in the reagent pad or conjugate pad when the dry stick is not used. When sample is added to the dry stick, the induced flow will transfer the labelled-control conjugate and the labelled-conjugate along the flow.

In a further embodiment, said base pad comprises a membrane; said membrane comprising said test line and said control line. In a further embodiment, said membrane is a nitrocellulose membrane. In one embodiment, one of the modules of the base pad is a membrane such as a nitrocellulose membrane. The nitrocellulose membrane has a porous structure, which makes it suitable for migration of liquid through capillary action. The membrane comprises two lines - a test line and a control line. Flow rates along the dry stick may be controlled by the characteristics of the membrane.

In a still further embodiment, said dry stick further comprises an absorbent pad. In the present context, the term "absorbent pad" refers to a material, which has the purpose of absorbing any liquid in excess when it has migrated through the base pad. Furthermore, backflow is prevented, which could cause incorrect results. Accordingly, the absorbent pad is arranged downstream of the test line and control line.

The material for the absorbent pad can be any material having great absorption characteristics such as a cellulose based material.

In an even further embodiment, said dry stick further comprises a backing card. In the present context, the term "backing card" refers to a material, which has no influence on the migration or on the reaction of the liquid sample or on reagent(s) or the agents capable of increasing the rate of the reaction. The backing card provides a stabilising basis for the dry stick and provides sufficient strength to maintain the desired physical shape and has substantially no interference with the production of a detectable signal. Thus, the backing card supports and stabilises at least a part of the base pad and potentially at least a part of the absorbent pad. In an embodiment of the present invention, the material for the backing card is selected from the group of polystyrene, vinyl and adhesive.

In a further embodiment, said dry stick further comprises a cover tape. In an even further embodiment, said cover tape covers at least said conjugated pad. In the present context, the term "cover tape" refers to a material, which has the purpose of making contact between the different membranes and/or pads. It has no chemical function but serves solely to apply pressure and contact between the different modules. The material could be any clear tape, where the adhesive does not have any influence flow of the dry stick.

In yet an embodiment of the present invention, the modules of the dry stick are in contact with one another by substantially fully overlapping, by partially overlapping or by laying adjacent to one another. In a further embodiment, said at least two modules partly overlaps. In an embodiment of the present invention the modules are overlapping by at least 5%, such as at least 10%, e.g. at least 25%, such as at least 50%, e.g. at least 75%, such as at least 80%, e.g. at least 90%, such as at least 95%. In the present context the term "substantially fully overlapping" relates to two separate modules being placed on top of one another. In the present context the term "partially overlapping" relates to two separate modules being overlapping with only part of the modules. A partial overlap of 100% relates to a full overlap and a deviation of 5% from the 100% full overlap relates to a substantially full overlap.

In an embodiment of the present invention the modules are laying adjacent to one another. This means that the pads are placed in contact with each other (touching each other). An overlap of 0% (but in contact) relates to the term "laying adjacent", furthermore, an overlap of less than 5% may be considered being within the term of "laying adjacent", such as an overlap of at the most 4%, e.g. an overlap of the most 3%, such as an overlap of the most 2% or e.g. an overlap of the most 1%.

In a preferred embodiment, the dry stick comprises a backing card and a base pad having three modules being a sample pad, a conjugate pad and a membrane having a control line downstream of the test line. The sample pad partially overlaps with the conjugate pad, which partially overlaps with the membrane. The dry stick furthermore comprises an absorbent pad downstream of the membrane and partially overlapping herewith.

In a further preferred embodiment, the dry stick comprises a backing card and a base pad having two modules being a reagent pad and a membrane having a control line downstream of the test line. The reagent pad partially overlaps with the membrane. The dry stick furthermore comprises an absorbent pad downstream of the membrane and partially overlapping herewith.

_Test line and control line_

The test line comprises a target analyte capable of binding to the labelled-conjugate, when not bound to haptoglobin. The control line comprises a control analyte capable of binding to the labelled-control conjugate. Accordingly, the control line is independent of the test line. In one embodiment, said control line is downstream of said test line. This is highly advantageous as it follows from the independency that the control line is to ensure that the stick is functioning and thus, the result obtained in the test line valid. When arranging the control line downstream of the test line, the functioning is tested after the test line and thus, it follows that the flow in the dry stick is correct at least until the control line is reached.

The wording "line" refers to the area where the target analyte and control analyte, respectively, are immobilized on the dry stick. This is often in the shape of a line, however, it may also be formed in a different geometrical pattern as long as the read-out of the binding to the target analyte and control analyte is feasible.

In the present context, "control analyte" is to be understood as the compound immobilized at the control line, which is capable of binding to the labelled-control conjugate. The control analyte is an immobilised control analyte. Hereby, is to be understood that the control analyte will remain at the control line even when said dry stick is in use.

The control analyte may be selected from the group of monoclonal antibodies, polyclonal antibodies, chimeric antibodies, nanobodies, aptamers and antibody mimicking proteins. In an even further embodiment, said control analyte is a third antibody.

In one embodiment, the concentration of the control analyte at the control line is 0.01-2 mg/ml, such as 0.02-1 mg/ml, like 0.05-0.5 mg/ml, such as around 0.2 mg/ml.

In the present context, "target analyte" is to be understood as the compound immobilized at the test line. The target analyte is capable of binding to the labelled-conjugate but not when the conjugate is in complex with haptoglobin. Accordingly, the target analyte may be haptoglobin or a part hereof as long as this part is able to bind to the labelled-conjugate in a competitive manner with the haptoglobin protein present in the milk sample. Alternatively, the target analyte may be a fusion protein comprising at least the part of haptoglobin binding to the labelled-conjugate. As an example, at least the part of haptoglobin binding to the labelled-conjugate or haptoglobin as such could be fused to bovine serum albumin (BSA). Alternatively, at least the part of haptoglobin may be biotinylated for immobilisation purposes.

The target analyte is an immobilised target analyte. Hereby, is to be understood that the target analyte will remain at the test line even when said dry stick is in use.

In one embodiment, the concentration of the target analyte at the test line is 0.01-2 mg/ml, such as 0.02-1 mg/ml, like 0.05-0.5 mg/ml, such as around 0.1 mg/ml.

In the present context, "complex" relates to a molecular entity formed by association between the labelled-conjugate in the base pad and the haptoglobin potentially present in the milk sample, which will form when the dry stick is in use i.e. when a sample is added to the dry stick and said sample comprises haptoglobin.

In one embodiment, the width of the control line and/or test line is 0.5-5 mm, such as 1-3 mm, like around 2 mm and the distance between the lines would be at least 0.5 mm, such as at least 1 mm in order to allow for sufficient distance between the lines. This would also allow for measurement of a background between the test line and control line to enable a more reliable measurement of the output of the test line and control line.

In a further embodiment, the target analyte and/or control analyte may be immobilized on the test line and/or control line, respectively by using blocking compounds such as polyvinylpyrrolidone (PVP), polyethylene glycol (PEG), bovine serum albumin (BSA) and salts like NaCl and KCl, to help bind the control analyte and/or target analyte to the base pad.

In addition, or alternatively, the target analyte and/or control analyte may be accompanied by pH-regulating agents such as Tris buffers, phosphate buffers and glycine buffers, for stabilising the pH and hence the secondary structure of the protein.

In a still further embodiment, surfactants may be included like Tween-20, Triton x-10, Plurionic, Tegretiol 20.

_Milk sample_

In the present context, "milk sample" is to be understood as a milk sample obtained directly from the animal after milking, where the sample has not yet been processed to consumer milk.

The animal is preferably a cow, but the invention may be used for other milk producing animals. "Animal" may be any arbitrary type of domesticated female milk producing and/or meat producing mammal, such as cow, goat, sheep, horse, camel, primate, dairy buffalo, donkey, yak, etc.

Advantageously, the dry stick may comprise a surfactant for the milk sample to provide optimal mix, release and line morphology when running the milk samples. In one embodiment, said base pad further comprises a surfactant. In a further embodiment said sample pad comprises said surfactant. In an even further embodiment, said surfactant is comprised in said reagent pad. In particular, the use of a surfactant in connection with the milk sample is beneficial with respect to the measurement of haptoglobin as the viscosity of the milk is usually increased for animals suffering from a disease.

In one embodiment, said surfactant is Pluronic F68, Pluronic F127, Surfactant 10G, Synperonic F108, Tergitol, Tween-20 and/or Triton X-100.

In a further embodiment, said surfactant is in a concentration in the range of 0.1-5 w/w%, such as 0.5-4 w/w%, like 1-3 w/w%, such as around 2 w/w%.

In an even further embodiment, said base pad comprises a pH-regulating agent. In a still further embodiment, said pH-regulating agent is a phosphate buffer, a borate buffer, a citric acid buffer and/or a Tris buffer. The pH-regulating agent ensures that the pH is maintained in the milk sample around a pH resembling that of the milk sample. In one embodiment, said milk sample has a pH in the range of 6-9. In a further embodiment, the milk sample has a pH in the range of 6.4-8.5. Hereby, the confirmation of the proteins in the milk sample will maintain as naturally occurring and allow for optimal binding of haptoglobin to the labelled-conjugate and/or optimal binding of the target analyte to the labelled-conjugate. Accordingly, pH is maintained optimal during the use of the dry stick.

In addition, further components may be added to the base pad and in particular to the conjugate pad in order to obtain a better release of the conjugate and the control-conjugate and validity of the results obtained when using the dry stick. Such compounds may be Sucrose and Trehalose. In one embodiment, these compounds are added in a concentration of 0.1-5 w/w%, such as 0.5-4 w/w%, like 1-3 w/w%, such as around 2 w/w% each.

Alternative or additionally to adding a surfactant to the base pad, sample pad and/or reagent pad, the milk sample may be mixed with a surfactant before providing it to the dry stick. In a further embodiment, said milk sample is mixed with a diluent comprising a surfactant, prior to applying said milk sample to said dry stick. In an even further embodiment, said surfactant is present in the diluent in a concentration of 0.1-4 w/w%, such as 0.5-2 w/w%, like around 1 w/w%.

In a still further embodiment, said surfactant is one or more surfactants selected from the following group: Pluronic F68, Pluronic F127, Surfactant 10G, Synperonic F108, Tergitol, Tween-20 and/or Triton X-100. In a still further embodiment, said surfactant is Tween-20.

Advantageously, the addition of a diluent increases the flowrate and mixing properties of the sample.

The diluent may beside water and surfactant comprise a pH regulating agent e.g. Tris-(hydroxymethyl)-methylamine), stabilizers e.g. sodium chloride, blocking agents e.g. PVP 40 and conservation agents e.g. ProClin 300. This would increase the reproducibility e.g. by blocking non-specific binding. Other pH regulating agents, stabilizers, blocking agents and conservation agents as commonly known to the skilled person may also be used.

The pH-regulating agent may be provided with the diluent. The concentration of the pH-regulating agent in the diluent is preferably in the range of 0.01-0.99 w/w%, such as 0.1-0.9 w/w%, like 0.25-0.75 w/w%, such as 0.35-0.65 w/w%, like around 0.5 w/w%.

*Conjugate*

In the present context, "labelled-conjugate" relates to the chemical substance that binds to haptoglobin in the milk or to the target analyte on the test line. The labelled-conjugate is a conjugate labelled with a label. Labelled-conjugate may relate to different labelled-conjugates such as two or more labelled-conjugates, such as three or more labelled-conjugates. However, in one embodiment, only one labelled-conjugate is present in the dry stick. The labelled conjugate may comprise a conjugate being selected from the group of monoclonal antibodies, polyclonal antibodies, chimeric antibodies, nanobodies, aptamers and antibody mimicking proteins. In one embodiment, said labelled-conjugate is a labelled first antibody.

In one embodiment, the concentration of the labelled-conjugate is 0.5-20 $\mu$g/ml, such as 1-15 $\mu$g/ml, like 2-12 $\mu$g/ml, such as 3-10 $\mu$g/ml, like 4-7 $\mu$g/ml, such as around 5 $\mu$g/ml.

In the present context, "labelled-control conjugate" relates to the chemical substance that binds to the control analyte at the control line. The labelled-control conjugate is a control conjugate labelled with a label. Labelled-control conjugate may relate to different labelled-control conjugates such as two or more labelled-control conjugates, such as three or more labelled-control conjugates. However, in one embodiment, only one labelled-control conjugate is present in the dry stick.

The labelled-control conjugate may comprise a control conjugate being selected from the group of monoclonal antibodies, polyclonal antibodies, chimeric antibodies, nanobodies, aptamers and antibody mimicking proteins. In a further embodiment, said labelled-control conjugate is a labelled second antibody.

The concentration of the control analyte in the control line is 1-30 μg/ml, such as 2-25 μg/ml, like 4-20 μg/ml, such as 5-15 μg/ml, like 7-12 μg/ml, such as around 10 μg/ml.

The labelled-control conjugate and the labelled-conjugate may be added to the conjugate pad by soaking, dip coating and spraying as commonly known to the skilled person in the art. This may be performed either manually or automatically.

The labelled-control conjugate and the labelled-conjugate are labelled with a label in order to be identified at the control line and the test line. The label would depend on the measuring system for the measurement of the labelled-control conjugate and labelled-conjugate present at the control line and test line, respectively. Thus, the labels may be chosen by the skilled person according to the wishes of the read-out. The label may be selected from the group of colloidal gold particles, latex particles, cellulose nanobeads, paramagnetic particles, radioactive particles and fluorescent particles as well as enzymes. In one embodiment, said labelled-control conjugate and/or said labelled-conjugate is labelled with colloidal gold particles.

Standardly, the three major groups of labels are: gold nanoparticles, latex particles and fluorescent particles.

The gold particles are gold particles often with a diameter around 40 nm. The particles are often called colloidal gold particles, since they are in a stable dispersion and require a specific pH and additives to the dispersion. These particles have a reddish/purple color. The small size gives a quick release and less issues with aggregation. However, sensitivity is often lower, since the color formation is lower per conjugate and thereby requires more conjugates. This is due to the smaller size and thereby the less reflection surface. The conjugation of conjugates to the gold particles is done passively, meaning it utilizes a combination of the electrostatic surface of the colloidal gold and hydrophobic interactions to bind the protein to the gold particles. As an example, it may be performed by mixing the conjugate and gold particles at a specified pH, adding excess bovine serum albumine and removing all unbound conjugate by centrifugation.

Latex particles are microspheres made of polyesters. These have a size of about 200-400 nm in diameter. They can be made in a variety of colors such as blue, green, purple and red. The particles are larger meaning less conjugate is required to create a visual color than for gold particles. Thus, less conjugate would be needed per dry stick, which could result in more variation and less flexibility. The bigger size may cause issues in relation to release and aggregation. The conjugate and label is normally covalently conjugated.

Fluorescent particles give high sensitivity and detect analytes at very low concentrations (pg/mL). A disadvantage is that it requires a specific light source for reading of the result but the advantages are improved sensitivity. There are multiple different types of fluorescent labels, such as organic dyes, metal-ligand complexes, fluorescent proteins, semiconductor quantum dots, lanthanide complexes, dye-doped polymer nanoparticles, fluorescent silica nanoparticles, xanthene dyes and cyanine dyes. In addition, fluorescence proteins can also be utilized. These different types of fluorescent dyes have different absorbance and emission wavelengths why it may be used for multiplexing.

The labels may be conjugated to the conjugate and/or control conjugate either passively or covalently.

## EXAMPLES

### Example 1 - Dry stick for haptoglobin measurement

In one embodiment, the dry stick 20 may be designed as disclosed in fig. 2A-B, where A shows a side view of the embodiment and B shows a top view. The dry stick 20 comprises a backing card 33 on which a sample pad 35, a conjugate pad 39, a membrane 41 and an absorbent pad 43 are arranged. Each of these different modules are partially overlapping to provide a smooth flow. The milk sample is added to the sample pad 35 and mixes with the conjugate (i.e. labelled-control conjugate and labelled-conjugate) in the conjugate pad 39 due to the downstream lateral flow 49 in the dry stick 20. Haptoglobin in the milk sample will bind to the conjugate to form a complex. Downstream of the conjugate pad 39 the membrane 41 comprises a test line 24 and a control line 25, which will bind to conjugate and control conjugate, respectively, from the conjugate pad 39. At the end of the run, the milk sample will be absorbed by the absorbent pad 43 at the end of the dry stick 20. Hereby, backflow is avoided along with contamination of the result. In addition, the dry stick will often hold a cover tape (not shown) to create pressure on all contact spots between the different materials and to ensure sufficient flow.

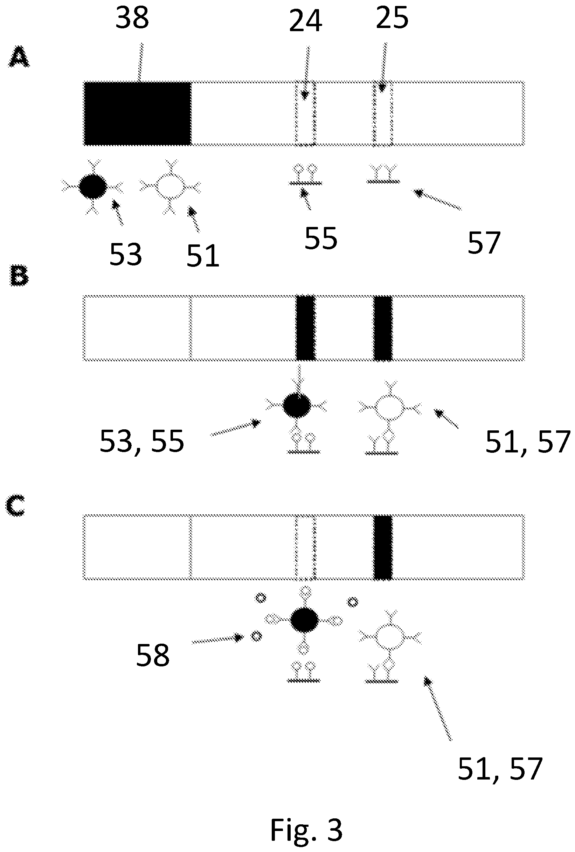

Figure 3A-C show one embodiment of a dry stick prior according to the invention. Figure 3A shows one embodiment of a dry stick prior to testing a milk sample. In this embodiment, the conjugate pad and the sample pad are shown as one module (i.e. reaction pad 38). The milk sample is introduced to the reaction pad 38, which comprises labelled-control conjugate 51 as well as labelled-conjugate 53. At the test line 24, target analyte 55 is present, while the control line 25 comprises control analyte 57.

If no haptoglobin is present in the milk sample, the labelled-conjugate 53 will bind to the target analyte 55 as demonstrated in figure 3B. In addition, the labelled-control conjugate 51 will bind to the control analyte 57. Hereby, a negative result is obtained being labelled both in the control line as well as the test line. In case of a positive result i.e. that haptoglobin 58 is present (shown in figure 3C) the haptoglobin will form a complex with the labelled-conjugate 53 prior to arriving at the test line 24 preventing the labelled-conjugate 53 from binding to the target analyte 55 at the test line 24. Nevertheless, the labelled-control conjugate will bind to the control analyte 57 at the control line 25. Thus, the dry stick will only be labelled at the control line 25. Depending on the concentration of haptoglobin in the milk, the labelled-conjugate 53 binds to the test line 24 correlating negatively (competitive) with the concentration haptoglobin. Accordingly, the intensity of the labelling at the test line 24 will vary depending on the amount of haptoglobin present in the milk sample.

The labelling at the control line 25 is independent of the amount of haptoglobin present in the milk sample and is a control of the lateral flow as such and the release of the conjugates from the conjugate pad. If the lateral flow has run satisfactorily and the conjugates been released as should be from the conjugate pad, a labelling will appear at the control line 25. If no labelling is registered at the control line 25, the result obtained by the dry stick cannot be trusted. It is thus advantageous for the control line to be present downstream of the test line for the results at the test line to be considered valid.

The control line may serve as a quality assurance indicating that the function of the dry stick is desired in the sense that if a control signal is produced, the dry stick is said to functions as desired.

However, it has been found that inter alia aging of a dry stick may influence the colour intensity produced by the test line and, if implemented, also the colour intensity of the control zone. Aging refers to the storage time of the dry stick and under which condition the dry stick has been stored. The colouring of the test line and control line is the result of the binding of the labelled-conjugate and labelled-control conjugate to the lines and the colour intensity is correlated with the amount of labelled conjugate and labelled-control conjugate which binds to the control line and test line respectively. Without being bound by theory, it is suggested that less labelled conjugate and less labelled-control conjugate are released with increasing time. In attempt to at least mitigate such aging effects, the invention suggests to use a ratio R between test signal and control signal as: *R=test signal/(test signal+control signal)*. This ratio *R* is in such cases correlated with the value of haptoglobin quantity and is found to be essentially independent on the amounts of labelled conjugate and labelled-control conjugate released from the conjugate pad by the milk applied to dry stick.

Reading of a dry stick may be implemented as described in e.g. WO2020/251460.

In preferred embodiments wherein the optical device 19 comprises a CCD-chip or a camera, such as a CCD camera, configured to optically read a colour intensity of the test line and optionally the control line. Such CCD-chip or camera provides a pixelated image of the test line and, optionally, the control line where each pixel represents a colour and its intensity. The test signal may be obtained in numerous manner, readily available to the skilled person, and may involve a spatial averaging or summation of the pixel values belonging the test and optionally the control line.

In accordance to this, the processor 13 may be configured to determine the test signal and optionally the control signal on the basis of a colour intensity of the test line and optionally across the control line.

Once the test signal and optionally the control signal has/have been provided the provided value of the haptoglobin quantity may be determined. The test signal is correlated with the value of haptoglobin quantity and a number of approaches are available to determine the value of haptoglobin quantity. A common denominator for some of the approaches is that a number of controlled experiments are carried out during which known and different amounts of haptoglobin are added to a plurality of known amounts of milk samples containing no blood, or if blood was present in these samples, it was in a concentration too low to be detected by the blood detection sensor device. Thereby, the amount of haptoglobin quantity is known for a plurality of milk samples. During these experiments, the haptoglobin sensor device is used to provide a value of haptoglobin in the milk samples. The thereby provided test signals are recorded and stored in a first database together with added amounts haptoglobin for each sample. Each such set of data may symbolically be written as *[Test signal; HP$_{added}$]* wherein *HP* refers to haptoglobin amount. If the control line is taken into account, the control signal may also be stored.

Upon providing values of haptoglobin quantity for milk sample with unknown value of haptoglobin quantity, the milk sample may be exposed to the haptoglobin sensor device providing a test signal. The provided value of haptoglobin quantity may then be derived through a database look-up in e.g. the database disclosed above storing corresponding values of test signal and optionally control signal and haptoglobin quantity. The actual value of haptoglobin quantity may be found by interpolation or extrapolation based on the data stored in the database.

Instead of using database, the data obtained by the experiments may be used in a regression analysis to provide e.g. a functional relationship between the data.

In preferred embodiments, the adjustment of the provided value of haptoglobin quantity is carried out on the basis of an mathematical model between the provided value of haptoglobin quantity and the provided value of blood quantity. Such a mathematical model may be a relatively simple algebraic model such as an equation taking as independent variables the provided value of haptoglobin quantity and provided value of blood quantity and providing as dependent variable the adjusted haptoglobin quantity, or it may be an artificial intelligence algorithm trained on data sets of known values of blood and haptoglobin quantities.

In other embodiment, the adjusted haptoglobin quantity is provided on the basis of a database look-up in a second data base storing corresponding values of haptoglobin quantity and blood quantity. It is noted, that as a database stores discrete values, interpolation within the stored values may increase the accuracy of the adjustment.

In a particular preferred embodiments, the mathematical model is an algebraic relationship comprising, a multiplier 22 for the provided value of haptoglobin quantity and an offset value 23. In order to increase the accuracy of the algebraic relationship, the value of the multiplier ($\alpha$) 22 and the offset value ($\beta$) 23 are both dependent on the provided value of blood quantity. This may symbolically be written as:

$$HP_{adjusted} = HP_{provided} * \alpha(blood\ quantity) + \beta(blood\ quantity)$$

The multiplier 22 and the offset value 23 are, in preferred embodiments, provided by the steps of

- providing a value of blood quantity by the blood detection sensor device 11, and
- providing a value of haptoglobin quantity by the haptoglobin sensor device 12 for a plurality of milk samples to which haptoglobin and blood are added in known quantities. This provides a number of sets of quantities where each set of quantities comprises a plurality of values of haptoglobin quantities associated with a unique value of blood quantity.

Based on this, a linear regression is carried out on one or more such as all of said sets of quantities to provide a number of multipliers and slopes each associated with a value of blood quantity.

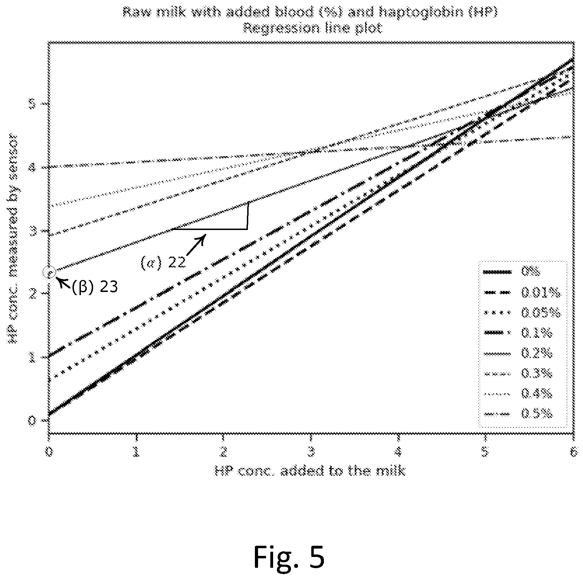

Experimental data provided by this method is disclosed in fig. 5.

While it may be advantageous to obtain an adjusted value of haptoglobin quantity, there may be situations where the quantity of blood in the milk is so high that no trustworthy haptoglobin measurement may be provided. In such and other situations, adjusting the provided value of haptoglobin quantity comprising discarding said provided value of haptoglobin quantity if said provided value of blood quantity is above an adjustment threshold. However, for values of blood quantity below the adjustment threshold, the adjustment may provide a trustworthy adjustment of the provided value of haptoglobin quantity and adjustment of the provided value of haptoglobin is preferably carried out by one or more of the methods disclosed herein.

In preferred embodiments, the blood detection sensor device comprises an RGB sensor device. The RGB sensor device has a light source configured to emit white light and a light receiver configured to receive scattered or transmitted light distinctly in the red, blue and green wave bands and being configured to detect blood in the said milk based on the received light. Such a RGB sensor device is disclosed in WO2014/055011 and the therein disclosed device can be used in connection with the present invention. Such a sensor device has the advantage of being capable of measuring in-line the amount of blood in milk.

It has been found that provided values of haptoglobin quantity may change during the time where the milk sample is in contact with haptoglobin sensor device 12. Without being bound by theory, some experiments suggest that if a high accuracy is aimed at, a relatively longer reaction time may be aimed at, whereas if a lesser accuracy can be accepted a shorter reaction time can be aimed at. The reaction time is the time during which the milk sample is in contact with the haptoglobin sensor device 12.

Thus, if no measures are taken and high accuracy is aimed at a dominating factor in measuring capacity will be the longest time needed to obtain the high accuracy. In many instances, it may be concluded that if the haptoglobin quantity provided is high, a high accuracy may not be needed (as the cow is very sick) whereas if the provided haptoglobin value is low, a high accuracy may be needed to reach a conclusion as to the health state of the cow. In the present invention, this has been implemented in some embodiments by using a haptoglobin threshold, where provided values of haptoglobin above the haptoglobin threshold is assigned to be determined values of haptoglobin quantities, which also may be referred to trustworthy values of haptoglobin quantities. Consequently, the reaction time is suggested to be increased if the haptoglobin sensor device provides values of haptoglobin quantities below the threshold.

While such strategies may be implemented by a user manually deciding a reaction time regime, the present invention suggests to apply a processor 13 comprised in the analysing unit to, inter alia, control the provision of the value of haptoglobin quantity.

To this, the processor 13 is in preferred embodiments, configured, on the basis of a reaction time (which is the time during which said milk sample has been in contact with the haptoglobin sensor device), to determine when the reaction time exceed a threshold time. The processor 13 being further configured to determine whether or not the provided value of haptoglobin quantity is above a haptoglobin threshold and in confirmative case assign the provided value of haptoglobin quantity to be a determined value of haptoglobin quantity. And, in non-confimative case the processor 13 increases the reaction time to a total reaction time and assign the provided value of haptoglobin quantity to be determined value of haptoglobin quantity at the point in time where the reaction time exceeds the total reaction time.

The progress over time of the provided value of haptoglobin quantity may be influenced by inter alia the environmental conditions to which the milk sample is exposed when in contact with the haptoglobin sensor 12 during the reaction time. If such influences are found to play an important role in the determination of the value of haptoglobin quantity, the milking arrangement may be equipped with an incubator 30 for incubating the milk sample applied to the haptoglobin sensor device 12. Such an incubator may typically comprise a void inside which the temperature and/or the humidity is/are controlled to be within selected ranges. In embodiments involving a dry stick, the dry stick is arranged in the incubator after milk is applied to it. The dry stick is then incubated for the threshold time 16 after which the above disclosed procedure is carried out pertaining to determine a value of haptoglobin quantity. It has been found that use of an incubator may reduce the reaction time in the sense that a trustworthy haptoglobin determination may be provided at a relatively shorter time when an incubator is used. This may turn into that the threshold time may be set relatively shorter than if no incubator is used.

When haptoglobin sensor device 12 comprises an optical device to read a test signal, the optical device may advantageously be arranged to read the dry stick while inside the incubator to eliminate the necessity to remove the dry stick from the incubator.

A milking arrangement according to the invention may advantageously be implemented in e.g. a herd management system where each animal, such as each cow, is uniquely identifiable e.g. by a tag containing an identification number unique for each animal. To this, the analysing unit is configured to digitally receive an identifier uniquely identifying a particular animal from which the milk sample is taken. This could for instance be provided by the milking arrangement comprising a reader configured to optical read a number provided e.g. on an ear tag placed on the animal or to electronically read e.g. an RIF tag arranged on or in the animal. When a haptoglobin quantity is determined, the processor is configured to digitally link the identifier and a determined haptoglobin quantity for the milk sample taken from said particular animal. The determined haptoglobin quantity together with the identifier are typically stored as a record in a database for later access. The processor 13 may also be configured to digitally output the linked identifier and determined haptoglobin quantity. This could for instance be output to a farmer and the output may be combined with an interpretation of the haptoglobin quantity allowing the farmer to e.g. contact a veterinarian.

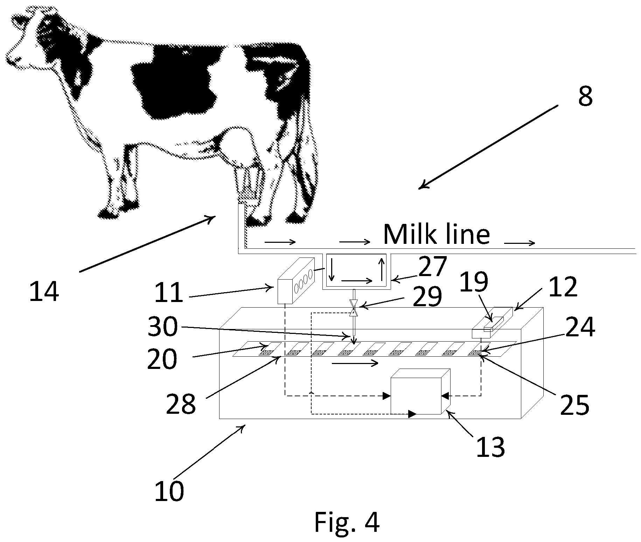

Fig. 4 schematically illustrates a milking arrangement according to another preferred embodiment of the invention. In fig. 4, dotted lines indicates signals, such as control signal or read-out signals from sensors. As illustrated, the milking arrangement comprising milking machine 14 including teat cups attached to the udder of a cow for milking milk out of the udder. The teat cups are connected to a milk line configured to feed harvest milk into a receptacle (not illustrated). A sampling line 27 is fluidicly connected to the milk line to sample milk from the milk flowing in the milk line. In fig. 4, the sampling line 27 is illustrated as bypass line with an electronically controlled valve 29. The sampling line 27, valve 29 and outlet 30 forms at least part of a sampling device.

The milking arrangement further comprises a conveyer 28 on which dry sticks 20 are placed and the conveyer conveys the dry sticks in a direction from left to right relatively to the orientation of fig. 4.

A blood detection sensor device 11 is arranged to detect the presence of blood in milk harvest by the milking machine. In the disclosed embodiment, the blood detection sensor device 11 is illustrated as an in-line device comprising an RGB sensor device. The RGB sensor device has a light source configured to emit white light and a light receiver configured to receive scattered or transmitted light distinctly in the red, blue and green wave bands and being configured to detect blood in the milk based on the received light. Such a RGB sensor device is disclosed in WO2014/055011 and the therein disclosed device can be used in connection with the present invention. It is to be noted, that the invention is not limited to such in-line blood detection sensor devices 11.

An outlet 30 downstream of the valve 29 is arranged in close proximity to the conveyer 29 so that when a dry stick is conveyed to a position below the outlet 30, opening of the valve 29 provides one or more drops of milk to be applied to the dry stick 20. In a position away from the outlet 30, a haptoglobin sensor device 12 is arranged. In the disclosed embodiment, the haptoglobin sensor device 12 comprising the dry stick 20 and an optical device 19 comprising a camera, which in the illustrated embodiment is a CCD camera, which is arranged to optically read the test signal, and if implemented, the control signal produced by the dry stick.

The milking arrangement comprises a processor 13. The processor 13 is configured to convert the signal(s) from the dry stick 20 (received from the haptoglobin sensor device 12) into a value of haptoglobin quantity in the milk sample. The processor 13 is configured to convert the signal received from the blood detection sensor into detection of blood in the milk sample. The processor 13 receives the signals from the sensors 11, 12 typically as electrical signals as illustrated by the dotted lines in fig. 4. The processor 13 is furthermore configured to control opening and closing of the valve 29 to allow milk to be applied to the dry stick 20. In addition, the processor is furthermore configured to control the movement of the conveyer 28 to advance dry sticks located on the conveyer 28 towards the outlet 30 providing milk and towards the CCD camera.

As can be readily understood, providing a distance between the position where the milk is applied to the dry stick and where the signal(s) produced by the dry stick is obtained by the CCD camera, represents a time during which the signal(s) can evolve on the dry stick(s). This time can be controlled by controlling the conveying speed, whereby the time during which the signal(s) evolves is controllable by the conveying speed. Thereby, if a certain amount of reaction time is aimed at, this may be accomplished by setting the conveying speed accordingly. However, it is to be emphasised that other ways of controlling the reaction time may be used. An alternative would be to arrange the CCD camera next to the outlet 30, typically with a field of view covering the position where the test line and the optional control line are located while the milk is applied to the dry stick. If longer incubation time is need and the dry stick has been moved past the field of view of the CCD camera, the conveyer may be reversed to move the stick backward to the reading position. Such positioning of the CCD camera furthermore has the advantage that the application of milk to the dry stick can be monitored which may be used to assure that milk is intentionally and correctly applied to the dry stick.

In the disclosed embodiment of fig. 4, the conveyer 20, the controller 13 and the haptoglobin sensor device 12 are illustrated as being contained in a physical delimited analysing unit 10, where the physical delimit is provided by a cabinet. By this, the interior of the cabinet may form an incubator where the atmosphere, such as humidity and/or temperature can be controlled.

Experimental results

Figure 5 illustrates experimental results obtained in connection with the present invention. "HP" in fig. 5 refers to haptoglobin. Data in figure 5, comes from experiments using milk samples. The milk samples are divided into 8 different subgroups where fresh blood was added in a known concentration ranging from 0-0.5%. Each milk-blood subgroup was further divided into 7 test tubes where a known amount of purified haptoglobin was added to the sample in concentrations ranging from 0-6 ng/ml. This gives a total of 56 sample combinations. Each sample combination was measured several times with the haptoglobin sensor device according to the present invention including a dry stick as disclosed in connection with figs. 2 and 3. The data illustrates the effect of blood in milk samples on the measured concentration of haptoglobin (test signal) in samples with a known haptoglobin concentration (HP added). The regression line for each blood concentration can be used to calibrate the results, to account for the effect of blood in milk, when the regression line is compared to the regression line for milk samples containing no blood. As disclosed above, this can be implemented as

$$HP_{adjusted} = HP_{provided} * \alpha(blood\ quantity) + \beta(blood\ quantity)$$

In fig. 5, the offset value 23 ($\beta$) and the multiplier 22 ($\alpha$) are indicated for the regression line of 0% added blood, and it can be seen that the offset value 23 is the value for the regression at 0 HP concentration added to the milk, and that the multiplier is slope of the regression line, which may be determined as:

$$\alpha = \frac{\Delta HP\ concetration\ measured\ by\ sensor}{\Delta HP\ concetration\ added\ to\ the\ milk}$$

List of reference symbols used

| 8  | Milking arrangement |
| 10 | Analysing unit |
| 11 | Blood detection sensor device |
| 12 | Haptoglobin sensor device |
| 13 | Processor |
| 14 | Milking machine |
| 19 | Optical device of the haptoglobin sensor device |
| 20 | Dry stick /Lateral flow assay |
| 22 | Slope |
| 23 | offset |
| 24 | Test line |
| 25 | Control line |
| 27 | Sampling line |
| 28 | Conveyor |
| 29 | Valve |
| 30 | Outlet |
| 33 | Backing card |
| 35 | Sample pad |
| 37 | Milk sample |
| 39 | Conjugate pad |

41 Membrane
43 Absorbent pad
49 Lateral flow
51 Labelled-control conjugate
53 labelled-conjugate
55 Target analyte
57 Specific binding means
58 Haptoglobin

**Claims**

1. A milking arrangement comprising a milking machine (14) for milking an animal, wherein the milking arrangement comprises

   • an analysing unit (10) configured to determining a value of haptoglobin quantity in a milk sample taken from milk harvest by the milking machine (14), said analysing unit (10) comprising

      • a blood detection sensor device (11) configured to detect the presence of blood in said milk harvest by the milking machine,
      • a haptoglobin sensor device (12) configured to provide a value of haptoglobin quantity in said milk sample, and
      • a processor (13);

   wherein the processor (13) being configured to adjusting said provided value of haptoglobin quantity based on the blood detection in the said milk.

2. A milking arrangement according to claim 1, wherein the processor (13) is configured to adjust said provided value of haptoglobin quantity by discarding said value of haptoglobin quantity if blood is detected in the said milk.

3. A milking arrangement according to any one of claim 1 or 2, wherein the blood detection sensor device (11) is configured to provide a value of blood quantity in said milk and said blood detection in the milk sample comprises said value of blood quantity in said milk.

4. A milking arrangement according to any one of the preceding claims, wherein the haptoglobin sensor device (12) comprising an optical device (19) configured to optically read

   • a test signal at a test line of a dry stick (20), and
   • optionally a control signal at a control line of said dry stick,
   and wherein
   • said processor (13) is configured to determine said provided value of haptoglobin quantity based on said test signal and optionally said control signal.

5. A milking arrangement according to claim 4, wherein the optical device (19) comprises a CCD-chip or a camera, such as a CCD-camera, configured to optically read a colour intensity of the test line and optionally the control line.

6. A milking arrangement according to any one of claims 4-5, wherein determine said provided value of haptoglobin quantity comprising a database look-up in a first database storing corresponding values of test signal and optionally control signal and haptoglobin quantity.

7. A milking arrangement according to any one of claims 4-6, wherein said dry stick (20) comprises:

   • a base pad capable of allowing lateral flow of fluid there through;

      • comprising a labelled-control conjugate and a labelled-conjugate diffusibly arranged herein, wherein said labelled-conjugate binds haptoglobin, and wherein a complex is formed between said labelled-conjugate and said haptoglobin when said dry stick is in use;

   wherein said base pad further comprises

• a test line comprising immobilised target analyte, wherein said immobilised target analyte binds to said labelled-conjugate when not in said complex; and
• a control line, which is spaced from said test line, and which comprises control analyte capable of binding to said labelled-control conjugate.

8. A milking arrangement according to claim 3, wherein adjusting said provided value of haptoglobin quantity is carried out on the basis of a mathematical model between the provided value of haptoglobin quantity and the provided value of blood quantity or on the basis of a database look-up in a second data base storing corresponding values of haptoglobin quantity and blood quantity, and wherein the adjusted value is the determined value of haptoglobin quantity in the milk sample.

9. A milking arrangement according to claim 8, wherein

• the mathematical model being an algebraic relationship comprises, a multiplier (22) for the provided value of haptoglobin quantity and an offset value (23), and
• the value of the multiplier (22) and the offset value (23) are both dependent on the provided value of blood quantity.

10. A milking arrangement according to claim 9, wherein the multiplier (22) and the offset value (23) are provided by

• providing a value of blood quantity by the blood detection sensor device (11), and
• providing a value of haptoglobin quantity by the haptoglobin sensor device (12) for a plurality of milk samples to which haptoglobin and blood are added in known quantities to provide a number of sets of quantities where each set of quantities comprises a plurality of values of haptoglobin quantities associated with a unique value of blood quantity, and
• performing a linear regression on one or more such as all of said sets of quantities to provide a number of multipliers and slopes each associated with a value of blood quantity.

11. A milking arrangement according to claim 3 or any one of claims 8-10, wherein adjusting said provided value of haptoglobin quantity comprising discarding said provided value of haptoglobin quantity if said provided value of blood quantity is above an adjustment threshold.

12. A milking arrangement according to any one of the preceding claims, wherein the blood detection sensor device comprises an RGB sensor device comprises a light source configured to emit white light and a light receiver configured to receive scattered or transmitted light distinctly in the red, blue and green wave bands and being configured to detect blood in the said milk based on the received light.

13. A milking arrangement according to any one of the preceding claims, wherein said processor (13) being further configured, on the basis of a reaction time being the time during which said milk sample has been in contact with the haptoglobin sensor device, to:

• determine when the reaction time exceed a threshold time, if the provided value of haptoglobin quantity is above a haptoglobin threshold, and

◦ in confirmative case assign the provided value of haptoglobin quantity to be a determined value of haptoglobin quantity, and
◦ in non-confimative case increase the reaction time to a total reaction time and assign the provided value of haptoglobin quantity to be determined value of haptoglobin quantity at the point in time where the reaction time exceeds the total reaction time.

14. A milking arrangement according to any one of the preceding claims, wherein the milking arrangement further comprising an incubator (30) for incubating said at least part of the milk sample applied to the haptoglobin sensor device (12).

15. A milking arrangement according to any one of the preceding claims, wherein analysing unit is configured to

• digitally receive an identifier uniquely identifying a particular animal from which the milk sample is taken,
• digitally link the identifier and the adjusted value of haptoglobin quantity for the milk sample taken from said particular animal, and
• digitally output the linked identifier and the adjusted value of haptoglobin quantity.

**Patentansprüche**

1. Melkanordnung, umfassend eine Melkmaschine (14) zum Melken eines Lebewesens, wobei die Melkanordnung umfasst

   • eine Analyseeinheit (10), die konfiguriert ist, um einen Wert einer Haptoglobinmenge in einer Milchprobe zu bestimmen, die von einem Milchertrag durch die Melkmaschine (14) entnommen wird, die Analyseeinheit (10) umfassend
   • eine Bluterkennungssensorvorrichtung (11), die konfiguriert ist, um das Vorhandensein von Blut in dem Milchertrag durch die Melkmaschine zu erkennen,
   • eine Haptoglobinsensorvorrichtung (12), die konfiguriert ist, um einen Wert der Haptoglobinmenge in der Milchprobe bereitzustellen, und
   • einen Prozessor (13);

   wobei der Prozessor (13) konfiguriert ist, um den bereitgestellten Wert der Haptoglobinmenge basierend auf der Bluterkennung in der Milch anzupassen.

2. Melkanordnung nach Anspruch 1, wobei der Prozessor (13) konfiguriert ist, um den bereitgestellten Wert der Haptoglobinmenge anzupassen, durch ein Verwerfen des Werts der Haptoglobinmenge, falls Blut in der Milch erkannt wird.

3. Melkanordnung nach einem der Ansprüche 1 oder 2, wobei die Bluterkennungssensorvorrichtung (11) konfiguriert ist, um einen Wert einer Blutmenge in der Milch bereitzustellen, und die Bluterkennung in der Milchprobe den Wert der Blutmenge in der Milch umfasst.

4. Melkanordnung nach einem der vorstehenden Ansprüche, die Haptoglobinsensorvorrichtung (12) umfassend eine optische Vorrichtung (19), die konfiguriert ist, um optisch zu lesen

   • ein Testsignal an einer Testlinie eines Trockenstreifens (20), und
   • optional ein Kontrollsignal an einer Kontrolllinie des Trockenstreifens, und wobei
   • der Prozessor (13) konfiguriert ist, um den bereitgestellten Wert der Haptoglobinmenge basierend auf dem Testsignal und optional dem Kontrollsignal zu bestimmen.

5. Melkanordnung nach Anspruch 4, wobei die optische Vorrichtung (19) einen CCD-Chip oder eine Kamera, wie etwa eine CCD-Kamera, umfasst, der/die konfiguriert ist, um eine Farbintensität der Testlinie und optional der Kontrolllinie optisch zu lesen.

6. Melkanordnung nach einem der Ansprüche 4 bis 5, das Bestimmen des bereitgestellten Werts der Haptoglobinmenge umfassend eine Datenbanksuche in einer ersten Datenbank, die entsprechende Werte des Testsignals und optional des Kontrollsignals und der Haptoglobinmenge speichert.

7. Melkanordnung nach einem der Ansprüche 4 bis 6, wobei der Trockenstreifen (20) umfasst:

   • eine Basenunterlage, die einen seitlichen Fluss von Fluid dahindurch ermöglicht;
   • umfassend ein markiertes Kontrollkonjugat und ein markiertes Konjugat, die hierin diffusionsfähig angeordnet sind, wobei das markierte Konjugat Haptoglobin bindet und wobei ein Komplex zwischen dem markierten Konjugat und dem Haptoglobin ausgebildet wird, wenn der Trockenstreifen in Verwendung ist;

   wobei die Basenunterlage ferner umfasst

   • eine Testlinie, umfassend einen immobilisierten Zielanalyten, wobei der immobilisierte Zielanalyt an das markierte Konjugat bindet, wenn nicht in dem Komplex; und
   • eine Kontrolllinie, die von der Testlinie beabstandet ist und die einen Kontrollanalyten umfasst, der in der Lage ist, an das markierte Kontrollkonjugat zu binden.

8. Melkanordnung nach Anspruch 3, wobei das Anpassen des bereitgestellten Werts der Haptoglobinmenge auf der Basis eines mathematischen Modells zwischen dem bereitgestellten Wert der Haptoglobinmenge und dem bereitgestellten Wert der Blutmenge oder auf der Basis einer Datenbanksuche in einer zweiten Datenbank ausgeführt wird, die entsprechende Werte der Haptoglobinmenge und der Blutmenge speichert, und wobei der angepasste Wert der bestimmte Wert der Haptoglobinmenge in der Milchprobe ist.

9. Melkanordnung nach Anspruch 8, wobei

• das mathematische Modell, das eine algebraische Beziehung ist, einen Multiplikator (22) für den bereitgestellten Wert der Haptoglobinmenge und einen Versatzwert (23) umfasst, und
• der Wert des Multiplikators (22) und der Versatzwert (23) beide abhängig von dem bereitgestellten Wert der Blutmenge sind.

10. Melkanordnung nach Anspruch 9, wobei der Multiplikator (22) und der Versatzwert (23) bereitgestellt werden durch

• Bereitstellen eines Werts der Blutmenge durch die Bluterkennungssensorvorrichtung (11), und
• Bereitstellen eines Werts der Haptoglobinmenge durch die Haptoglobinsensorvorrichtung (12) für eine Vielzahl von Milchproben, denen Haptoglobin und Blut in bekannten Mengen zugesetzt werden, um eine Anzahl von Mengensätzen bereitzustellen, wobei jeder Mengensatz eine Vielzahl von Werten der Haptoglobinmengen umfasst, die einem eindeutigen Wert der Blutmenge zugeordnet sind, und
• Durchführen einer linearen Regression an einem oder mehreren, wie allen, der Mengensätze, um eine Anzahl von Multiplikatoren und Steigungen bereitzustellen, die jeweils einem Wert der Blutmenge zugeordnet sind.

11. Melkanordnung nach Anspruch 3 oder einem der Ansprüche 8 bis 10, das Anpassen des bereitgestellten Werts der Haptoglobinmenge umfassend das Verwerfen des bereitgestellten Werts der Haptoglobinmenge, falls der bereitgestellte Wert der Blutmenge über einer Anpassungsschwelle liegt.

12. Melkanordnung nach einem der vorstehenden Ansprüche, wobei die Bluterkennungssensorvorrichtung eine RGB-Sensorvorrichtung umfasst, die eine Lichtquelle, die konfiguriert ist, um weißes Licht auszusenden, und einen Lichtempfänger umfasst, der konfiguriert ist, um gestreutes oder durchgelassenes Licht deutlich in den roten, blauen und grünen Wellenbändern zu empfangen, und der konfiguriert ist, um Blut in der Milch basierend auf dem empfangenen Licht zu erkennen.

13. Melkanordnung nach einem der vorstehenden Ansprüche, wobei der Prozessor (13), auf der Basis einer Reaktionszeit, die die Zeit ist, während der die Milchprobe mit der Haptoglobinsensorvorrichtung in Kontakt war, ferner konfiguriert ist zum:

• Bestimmen, wann die Reaktionszeit eine Schwellenzeit überschreitet, falls der bereitgestellte Wert der Haptoglobinmenge über einer Haptoglobinschwelle liegt, und

o in einem bestätigenden Fall, Zuweisen des bereitgestellten Werts der Haptoglobinmenge, ein bestimmter Wert der Haptoglobinmenge zu sein und
o in einem nicht bestätigenden Fall, Erhöhen der Reaktionszeit zu einer Gesamtreaktionszeit und Zuweisen des bereitgestellten Werts der Haptoglobinmenge, bestimmter Wert der Haptoglobinmenge zu dem Zeitpunkt zu sein, an dem die Reaktionszeit die Gesamtreaktionszeit überschreitet.

14. Melkanordnung nach einem der vorstehenden Ansprüche, die Melkanordnung ferner umfassend einen Inkubator (30) zum Inkubieren des mindestens einen Teils der Milchprobe umfasst, die auf die Haptoglobinsensorvorrichtung (12) aufgebracht wird.

15. Melkanordnung nach einem der vorstehenden Ansprüche, wobei die Analyseeinheit konfiguriert ist zum

• digitalen Empfangen einer Kennung, die ein spezifisches Tier, von dem die Milchprobe entnommen wird, eindeutig identifiziert,
• digitales Verknüpfen der Kennung und des angepassten Werts der Haptoglobinmenge für die Milchprobe, die von dem spezifischen Tier entnommen wird, und
• digitales Ausgeben der verknüpften Kennung und des angepassten Werts der Haptoglobinmenge.

**Revendications**

1.  Agencement de traite comprenant une machine à traire (14) permettant de traire un animal, dans lequel l'agencement de traite comprend

    • une unité d'analyse (10) configurée pour déterminer une valeur de quantité d'haptoglobine dans un échantillon de lait prélevé sur du lait récolté par la machine à traire (14), ladite unité d'analyse (10) comprenant
    • un dispositif capteur de détection de sang (11) configuré pour détecter la présence de sang dans ledit lait récolté par la machine à traire,
    • un dispositif capteur d'haptoglobine (12) configuré pour fournir une valeur de quantité d'haptoglobine dans ledit échantillon de lait, et
    • un processeur (13) ;

    dans lequel le processeur (13) est configuré pour ajuster ladite valeur fournie de quantité d'haptoglobine sur la base de la détection de sang dans ledit lait.

2.  Agencement de traite selon la revendication 1, dans lequel le processeur (13) est configuré pour ajuster ladite valeur fournie de quantité d'haptoglobine en écartant ladite valeur de quantité d'haptoglobine si du sang est détecté dans ledit lait.

3.  Agencement de traite selon l'une quelconque des revendications 1 ou 2, dans lequel le dispositif capteur de détection de sang (11) est configuré pour fournir une valeur de quantité de sang dans ledit lait et ladite détection de sang dans l'échantillon de lait comprend ladite valeur de quantité de sang dans ledit lait.

4.  Agencement de traite selon l'une quelconque des revendications précédentes, dans lequel le dispositif capteur d'haptoglobine (12) comprend un dispositif optique (19) configuré pour lire optiquement

    • un signal de test sur une ligne de test d'un bâtonnet sec (20), et
    • facultativement un signal témoin sur une ligne témoin dudit bâtonnet sec, et dans lequel
    • ledit processeur (13) est configuré pour déterminer ladite valeur fournie de quantité d'haptoglobine sur la base dudit signal de test et facultativement dudit signal témoin.

5.  Agencement de traite selon la revendication 4, dans lequel le dispositif optique (19) comprend une puce CCD ou une caméra, telle qu'une caméra CCD, configurée pour lire optiquement une intensité de couleur de la ligne de test et facultativement de la ligne témoin.

6.  Agencement de traite selon l'une quelconque des revendications 4 à 5, dans lequel la détermination de ladite valeur fournie de quantité d'haptoglobine comprend une recherche dans une base de données dans une première base de données stockant des valeurs correspondantes de signal de test et, facultativement, de signal témoin et de quantité d'haptoglobine.

7.  Agencement de traite selon l'une quelconque des revendications 4 à 6, dans lequel ledit bâtonnet sec (20) comprend :

    • un tampon de base capable de permettre un écoulement latéral de fluide à travers celui-ci ;
    • comprenant un conjugué témoin marqué et un conjugué marqué disposé de manière diffusible dans celui-ci, dans lequel ledit conjugué marqué se lie à l'haptoglobine, et dans lequel un complexe se forme entre ledit conjugué marqué et ladite haptoglobine lorsque ledit bâtonnet sec est utilisé ;

    dans lequel ledit tampon de base comprend en outre

    • une ligne de test comprenant un analyte cible immobilisé, dans lequel ledit analyte cible immobilisé se lie audit conjugué marqué lorsqu'il n'est pas dans ledit complexe ; et
    • une ligne témoin, qui est espacée de ladite ligne de test, et qui comprend un analyte témoin capable de se lier audit conjugué témoin marqué.

8.  Agencement de traite selon la revendication 3, dans lequel l'ajustement de ladite valeur fournie de quantité d'haptoglobine est effectué sur la base d'un modèle mathématique entre la valeur fournie de quantité d'haptoglobine et la valeur fournie de quantité de sang ou sur la base d'une recherche dans une base de données dans une seconde base de données stockant des valeurs correspondantes de quantité d'haptoglobine et de quantité de sang, et dans lequel la valeur ajustée est la valeur déterminée de quantité d'haptoglobine dans l'échantillon de lait.

9. Agencement de traite selon la revendication 8, dans lequel

   • le modèle mathématique qui est une relation algébrique comprend un multiplicateur (22) pour la valeur fournie de quantité d'haptoglobine et une valeur de décalage (23), et
   • la valeur du multiplicateur (22) et la valeur de décalage (23) dépendent toutes deux de la valeur fournie de quantité de sang.

10. Agencement de traite selon la revendication 9, dans lequel le multiplicateur (22) et la valeur de décalage (23) sont fournis par

   • la fourniture d'une valeur de quantité de sang par le dispositif capteur de détection de sang (11), et
   • la fourniture d'une valeur de quantité d'haptoglobine par le dispositif capteur d'haptoglobine (12) pour une pluralité d'échantillons de lait auxquels de l'haptoglobine et du sang sont ajoutés en quantités connues pour fournir un certain nombre d'ensembles de quantités où chaque ensemble de quantités comprend une pluralité de valeurs de quantités d'haptoglobine associées à une valeur unique de quantité de sang, et
   • la réalisation d'une régression linéaire sur un ou plusieurs, par exemple l'ensemble, desdits ensembles de quantités afin de fournir un certain nombre de multiplicateurs et de pentes associés chacun à une valeur de quantité de sang.

11. Agencement de traite selon la revendication 3 ou l'une quelconque des revendications 8 à 10, dans lequel l'ajustement de ladite valeur fournie de quantité d'haptoglobine comprend le fait d'écarter ladite valeur fournie de quantité d'haptoglobine si ladite valeur fournie de quantité de sang est supérieure à un seuil d'ajustement.

12. Agencement de traite selon l'une quelconque des revendications précédentes, dans lequel le dispositif capteur de détection de sang comprend un dispositif capteur **RVB** comprenant une source lumineuse configurée pour émettre une lumière blanche et un récepteur de lumière configuré pour recevoir une lumière diffusée ou transmise distinctement dans les bandes d'ondes rouge, bleue et verte, et configuré pour détecter du sang dans ledit lait sur la base de la lumière reçue.

13. Agencement de traite selon l'une quelconque des revendications précédentes, dans lequel ledit processeur (13) est en outre configuré, sur la base d'un temps de réaction qui est le temps pendant lequel ledit échantillon de lait a été en contact avec le dispositif capteur d'haptoglobine, pour :

   • déterminer, lorsque le temps de réaction dépasse un temps seuil, si la valeur fournie de quantité d'haptoglobine est supérieure à un seuil d'haptoglobine, et

       o en cas de confirmation, attribuer la valeur fournie de quantité d'haptoglobine à une valeur déterminée de quantité d'haptoglobine, et
       o en cas de non-confirmation, augmenter le temps de réaction jusqu'à un temps de réaction total et attribuer la valeur fournie de quantité d'haptoglobine à déterminer la valeur de quantité d'haptoglobine au moment où le temps de réaction dépasse le temps de réaction total.

14. Agencement de traite selon l'une quelconque des revendications précédentes, dans lequel l'agencement de traite comprend en outre un incubateur (30) permettant d'incuber ladite au moins une partie de l'échantillon de lait appliquée au dispositif capteur d'haptoglobine (12).

15. Agencement de traite selon l'une quelconque des revendications précédentes, dans lequel l'unité d'analyse est configurée pour

   • recevoir numériquement un identifiant qui identifie de manière unique un animal particulier sur lequel l'échantillon de lait est prélevé,
   • lier numériquement l'identifiant et la valeur ajustée de quantité d'haptoglobine pour l'échantillon de lait prélevé sur ledit animal particulier, et
   • émettre numériquement l'identifiant lié et la valeur ajustée de quantité d'haptoglobine.

Fig. 1

Fig. 2

Fig. 3

8

14

Milk line

27

11

29

19

12

30

24

20

28

25

13

10

Fig. 4

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018180632 A1
- WO 2020251457 A
- WO 2020251460 A
- WO 2014055011 A